# EUROPEAN PATENT SPECIFICATION

(11) **EP 1 968 699 B1**
(45) Date of publication and mention of the grant of the patent: **14.04.2010**
(21) Application number: 06845327.3
(22) Date of filing: 11.12.2006
(51) Int. Cl.: A61N 1/372, A61N 1/08, G06F 19/00, H04L 29/06

(54) **PROVIDING A SECURE FEATURE SET DISTRIBUTION INFRASTRUCTURE FOR MEDICAL DEVICE MANAGEMENT**
BEREITSTELLUNG EINER SICHERHEITSMERKMAL-SET-VERTEILUNGSINFRASTRUKTUR FÜR MEDIZINPRODUKTE-MANAGEMENT
FOURNITURE D'INFRASTRUCTURE DE DISTRIBUTION D'ENSEMBLE DE CARACTERISTIQUES SECURISEE POUR GESTION DE DISPOSITIFS MEDICAUX

(30) Priority: 12.12.2005 US 299980
(43) Date of publication of application: 17.09.2008
(73) Proprietor: CARDIAC PACEMAKERS, INC., St. Paul, Minnesota 55112-5798 (US)
(72) Inventor: SMYTHE, Alan, H., White Bear Lake, MN 55110 (US); SIMMS, Howard, D., Shoreview, MN 55126 (US); HOYME, Kenneth, P., Plymouth, MN 55446 (US); JELATIS, George, D., Minneapolis, MN 55417-1429 (US)
(74) Representative: Charig, Raymond Julian
(86) International application number: PCT/US2006/047493
(87) International publication number: WO 2007/070552

(56) References cited:
- US-A- 2005 240 246
- US-A1- 2001 027 331
- US-A1- 2003 046 677

## Description

### TECHNICAL FIELD

The present invention relates in general to medical device management and, specifically, to a system and method for providing a secure feature set distribution infrastructure for medical device management.

### BACKGROUND ART

Cardiac implantable medical devices (IMDs), such as pacemakers and implantable cardioverter-defibrillators (ICDs), are generally implanted subdermally over the pectoralis major muscle. A set of leads to deliver cardiac therapy and monitor cardiopulmonary physiology is also implanted transvenously under local anesthesia using either the cephalic and subclavian veins. Power for IMDs is provided conventionally by batteries that have high-energy density, low internal loss, and long shelf life. For example, implanted single-chamber pacemakers use lithium iodine batteries and have an expected implant life of seven to twelve years. Dual-chamber pacemakers use lithium silver vanadium oxide batteries and have an expected implant life of five to ten years.

Ordinarily, an entire IMD is replaced when the battery life has expired to take advantage of new features and advances in technologies that may have occurred since the time of original implant. Replacement of an IMD requires surgery, which is accompanied by attendant risks of injury, infection, recovery time, and related complications. Surgical risk can be minimized by limiting or eliminating the situations in which a device must be replaced, such as upon the occurrence of a broken or failing lead or problematic IMD.

Prior to replacement, interim upgrades to the operational characteristics and programming of an IMD can be performed in-clinic by upgrading on-board programming software or firmware using a programmer-type device. These types of updates are limited to a clinical setting and require a physician to be present, which can be problematic if minor yet necessary upgrades need to be performed to a large patient population. Modifications must be precisely matched to the specific model and software or firmware revision level of each IMD. Ensuring correct upgradeability requires extra caution to avoid introducing changes that could harm or render the device inoperable, thereby requiring possible early replacement.

When available, in-clinic software or firmware upgrades can only be performed under the supervision of a physician. A programmer-type device is used to interrogate the IMD through inductive telemetry. Due to the close proximity of the physician to the patient, authorization is implied and secure exclusive access to the IMD assumed. Software or firmware upgrades are limited to only the device implanted in that patient. Other medical devices, whether implanted or external, must be interrogated and upgraded separately. As a result, managing multiple medical devices requires individually tracking each medical device and the associated operating characteristics for functional upgrades and on-going maintenance on a patient-by-patient basis. This medical device management burden is exacerbated by a large patient population.

Therefore, there is a need for a medical device management system providing remote, non-surgical upgrades to IMDs. Preferably, such an approach would provide non-clinical and secure, authenticated upgrades to software and firmware used in both implantable and external medical devices on per patient and patient population bases. Such an approach would preferably leverage public infrastructure, such as the Internet, to provide the most economical solution to managing medical devices, while using cryptographic technology to maintain a high level of security and reliability.

US 2001/0027331 describes a system in which data from an implantable medical device and a data center can be transferred based on a differentiated encryption system that allows for the differentiation, segregation and classification of data at required levels of security. US 2005/0240246 describes a communication system for communication between a deployed implantable medical device and a large scale computer. Transmissions between the IMDs and the computer are encrypted and/or authenticated end-to-end. US 2003/0046677 describes a system for identifying software currently operating in an implantable medical device system and updating it if required.

### DISCLOSURE OF THE INVENTION

A system and method includes a secure distribution server maintaining a configuration catalog of unique mappings between a patient management device and one or more associated patient medical devices, including passive and active implantable and external medical devices. Identification of the software and firmware provided on each associated patient medical device is either periodically requested by the patient management device or autonomously reported to the patient management device by each device. In one embodiment, the patient management device requests updates to the software and firmware of the devices and of the patient management device itself from the secure distribution server on a periodic basis and the secure distribution server provides any new or modified sets of features as update packages, which are either already digitally signed by a trusted source or are digitally signed by the secure distribution server for a specific patient management device. In a further embodiment, the secure distribution server periodically provides any new or modified feature sets to the patient management device as such sets become available. The patient management device authenticates the trusted source and checks the integrity of each update package prior to installation. The digital signing by the trusted source is combined with signature verification at each patient management device to ensure the authenticity and integrity of the update package; these processes provide a chain of trust to securely distribute the new or modified feature sets. The patient management device sends a notification back to the secure distribution server upon successful upgrade or installation. In a further embodiment, each device, rather than the patient management device, does performs signature verification of each update package prior to installation to extend the chain of trust to the device itself. Accordingly, both minor and wholesale changes to software and firmware can be distributed to remote devices over one or more networks without the need for an in-clinic patient visit.

One embodiment provides a system and method for providing a secure feature set distribution infrastructure for medical device management. A unique association is mapped for data download between a medical device and a communications device transiently coupleable to the medical device. A configuration catalog is maintained, including operational characteristics of at least one of the medical device and the communications device. The operational characteristics as maintained in the configuration catalog are periodically checked against a database storing downloadable sets of features and one or more feature sets including changed operational characteristics are identified for distribution. The one or more feature sets are digitally signed and the one or more feature sets are provided to the communications device over a plurality of networks. The one or more feature sets are authenticated and their integrity is checked over a chain of trust originating with a trusted source and terminating at the communications device.

Still other embodiments of the present invention will become readily apparent to those skilled in the art from the following detailed description, wherein are described embodiments of the invention by way of illustrating the best mode contemplated for carrying out the invention. As will be realized, the invention is capable of other and different embodiments and its several details are capable of modifications in various obvious respects. Accordingly, the drawings and detailed description are to be regarded as illustrative in nature and not as restrictive.

### DESCRIPTION OF THE DRAWINGS

FIGURE 1 is a functional block diagram showing a system for providing a secure feature set distribution infrastructure for medical device management in accordance with one embodiment.
FIGURE 2 is a data structure diagram showing, by way of example, a configuration catalog for storing medical device mappings.
FIGURE 3 is a data structure diagram showing, by way of example, an update package for providing an updated feature set.
FIGURE 4 is a block diagram showing the secure distribution server of FIGURE 1.
FIGURES 5A-B are routing diagrams showing end-to-end secure package processing by the system of FIGURE 1.
FIGURE 6 is a process flow diagram showing a configuration catalog update dialogue performed by the system of FIGURE 1.
FIGURE 7 is a process flow diagram showing an upload dialogue performed by the system of FIGURE 1.
FIGURE 8 is a flow diagram showing a server method for providing a secure feature set distribution infrastructure for medical device management in accordance with one embodiment.
FIGURE 9 is a flow diagram showing a routine for periodically retrieving data for use in the method of FIGURE 7.
FIGURE 10 is a flow diagram showing a routine for processing an update request for use in the method of FIGURE 7.
FIGURE 11 is a flow diagram showing a method for providing a secure feature set distribution infrastructure for medical device management in accordance with one embodiment.
FIGURE 12 is a flow diagram showing a routine for performing a periodic update for use in the method of FIGURE 11.
FIGURE 13 is a flow diagram showing a routine for sending stored data for use in the method of FIGURE 11.

### BEST MODE FOR CARRYING OUT THE INVENTION

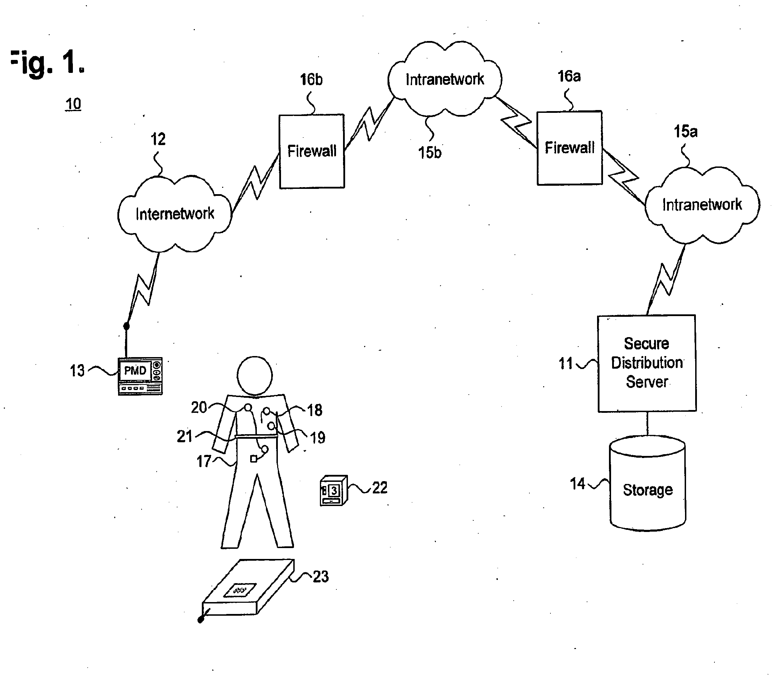

FIGURE 1 is a functional block diagram showing a system 10 for providing a secure feature set distribution infrastructure for medical device management in accordance with one embodiment. The system 10 includes a secure distribution server 11 and patient management device 13 that is remotely interconnected via a plurality of networks, including an internetwork 12, such as the Internet, and intranetworks 15a, 15b. In one embodiment, each individual network is securely protected at each border by a firewall 16a, 16b, gateway, or similar security device. Each firewall 16a, 16b protects an associated network against unauthorized access and intrusion. Other topologies, configurations, and arrangements of networks are possible.

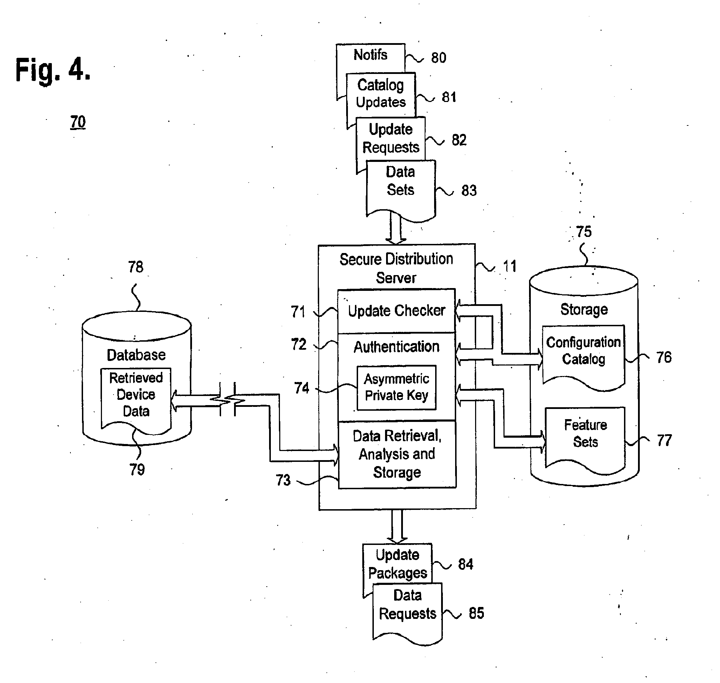

The secure distribution server 11 is operatively coupled to a storage device 14 and is remotely accessible by the patient management device 13 over the plurality of networks to securely distribute updates or new feature sets, as further described below with reference to FIGURE 4. The patient management device 13 functions primarily as a communications device and executes a set of software modules defined as patient communication application software. In addition, the patient management device 13 can also include medical device functionality. The patient management device 13 includes user interfacing means, including a speaker, microphone, display, interactive user interface, such as a touch screen or keypad, and a secure wireless interface, such as provided by "strong" Bluetooth, wireless fidelity "WiFi" or "WiMax," or other radio frequency interfaces, to allow external and implantable medical devices to be logically interfaced. In one embodiment, the patient management device 13 is implemented as a dedicated hardware device specifically interfacing to external and implantable medical devices. In a further embodiment, the patient management device 13 could be implemented integral to or as an add-on module functionally coupled to a portable computing device, such as a personal digital assistant, cellular telephone, and similar devices.

Interfaceable external and implantable medical devices include active therapeutic or monitoring devices, such as an implantable medical device 18, implantable sensor 19, external medical device 20, or external sensor 21, and passive therapeutic or monitoring devices, such as external medical device 22 and external sensor 23. These therapeutic and monitoring devices can deliver therapy or provide sensor readings that can be processed by the secure distribution server 11 or similar device into quantitative, physiological measures. Implantable medical devices 18 include pacemakers, implantable cardioverter-defibrillators, cardiac resynchronization devices, drug delivery devices, and neurological implants. Implantable sensors 19 include heart or respiratory monitors, and posture, activity, or blood chemistry monitors. Active external medical devices 20 include automated external defibrillators. Active external sensors 21 include Holter monitors. Passive external medical devices 22 include pill dispensers. Finally, passive external sensors 23 include weight scales and blood pressure monitors. Other types of implantable medical devices, implantable sensors, external medical devices, and external sensors, active as well as passive, are possible.

Operationally, the secure distribution server 11 maintains a configuration catalog of operational characteristics of the patient management device 13 and the one or more associated medical devices 18-23. The operational characteristics of the devices are either requested by the patient management device 13 from each device or are periodically reported to the patient management device 13 by each device. The configuration catalog stores a unique association between the patient management device 13 and each medical device for each patient 17. In one embodiment, the patient management device 13 periodically checks for updates or new feature sets stored as program code by the secure distribution server 11 and then the patient management device securely downloads or "pulls" any modified or new firmware or software, referred to as "updates," as secure packages. Each secure package is either stored on the secure distribution server in digitally signed form, that is, signed by another trusted source, or can be digitally signed by the secure distribution server for a specific patient management device. In a further embodiment, the secure distribution server 11 on-demand or incrementally sends or "pushes" the program code for any modified or new firmware or software to the patient management device 13 as such updates become available or by unilaterally broadcasting the updates to a certain class of devices, such as patient management devices. An on-demand update can be initiated by either the secure distribution server 11 or via an authenticated client on the internetwork 12 or similar device. Upon authenticating and checking the integrity of each update package, the patient management device 13 installs the updated or new feature set on the appropriate medical device and notifies the secure distribution server 11 upon successful completion. In a further embodiment, one or more of the medical devices, rather than the patient management device 13, authenticate and integrity check each update package prior to installation. Additionally, the secure distribution server 11 or similar device periodically retrieves stored data from the patient management device 13, which was previously collected from the one or more associated medical devices. The medical device mappings configuration catalog and update packages will now be described.

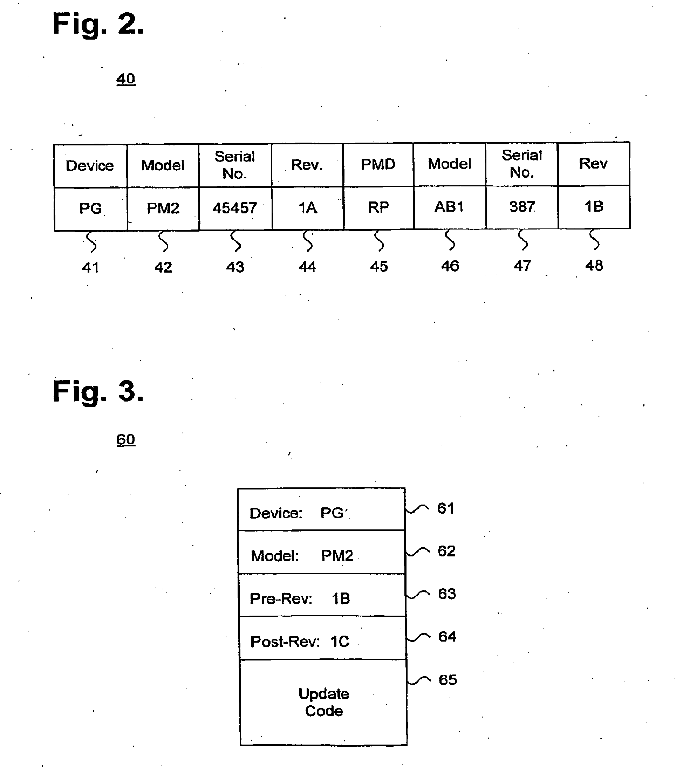

FIGURE 2 is a data structure diagram showing, by way of example, a configuration catalog 40 for storing medical device mappings. The configuration catalog 40 serves two purposes. First, the configuration catalog 40 maps the unique association between a patient management device 13 and a particular medical device, such as IMD 18. Second, the configuration catalog 40 records the operational characteristics of both the patient management device 13 and each associated medical device. For instance, an entry can identify the medical device by type 41, model 42, serial number 43, and software revision level 44. Similarly, the same entry, or a separate linked entry (not shown), can include the patient management device type 45, model 46, serial number 47, and software revision level 48. Other types of configuration catalog and record structures and arrangements are possible.

The operational characteristics recorded in the configuration catalog 40 can be provided initially by the manufacturer of each device and the patient management device 13. Subsequently, in one embodiment, the patient management device 13 periodically polls each device to determine current operational characteristics and those operational characteristics, plus operational characteristics of the patient management device 13, are reported to the secure distribution server 11 to update the configuration catalog 40. In a further embodiment, the devices periodically report their operational characteristics to the patient management device 13, which are then reported to the secure distribution server 11 for configuration catalog update. Other forms of configuration catalog updating are possible.

FIGURE 3 is a data structure diagram showing, by way of example, an update package 60 for providing an updated feature set. An update package 60 is generated by the secure distribution server 11 to securely distribute modified or new sets of features for a patient management device or one or more associated medical devices. In a further embodiment, an update package 60 can contain a set of "atomic" patches for features that must either be installed as a complete non-divisible set, or not installed at all. In one embodiment, each update package 60 is provided to a patient management device 13 in response to an update request. In a further embodiment, each update package 60 is on-demand or incrementally provided to a patient management device 13 as updated features become available or by unilaterally broadcasting the updated features to a certain class of devices, such as patient management devices. Other forms of secure update package distribution are possible.

Each update package 60 includes a header that identifies the device to which the update code 65 applies, such as device type 61 and model 62. In addition, the header identifies the pre-updating software revision level 63 and post-updating software revision level 64, which respectively identify the software revision levels for the update to apply and at which the device will be after the update is installed. In a further embodiment, the pre-updating software revision level 63 can specify a range of pre-updating patch revision levels, or just a single pre-updating patch revision level. The update package 60 is encapsulated within a digitally signed "envelope" (not shown) or package created by the secure distribution server 11. The update package 60 can either be pre-digitally-signed by a trusted source, such as by the manufacturer, or can be digitally signed by the secure distribution server for a specific patient management device. In one embodiment, update package authentication is provided through a form of asymmetric encryption, such as public/private key-pair based digital signatures, although other types of authentication and encryption are possible.

FIGURE 4 is a block diagram showing the secure distribution server 11 of FIGURE 1. The secure distribution server 11 serves as a focal point for securely distributing modified and new feature sets 77 to patient management devices and associated medical devices. The secure distribution server 11 executes a set of software modules defined as secure distribution server software. The secure distribution server 11 accesses the feature sets 77 through a secure storage device 75, along with a configuration catalog 76 that maps the unique associations between the patient management device 13 and one of possibly several medical devices for a particular patient 17.

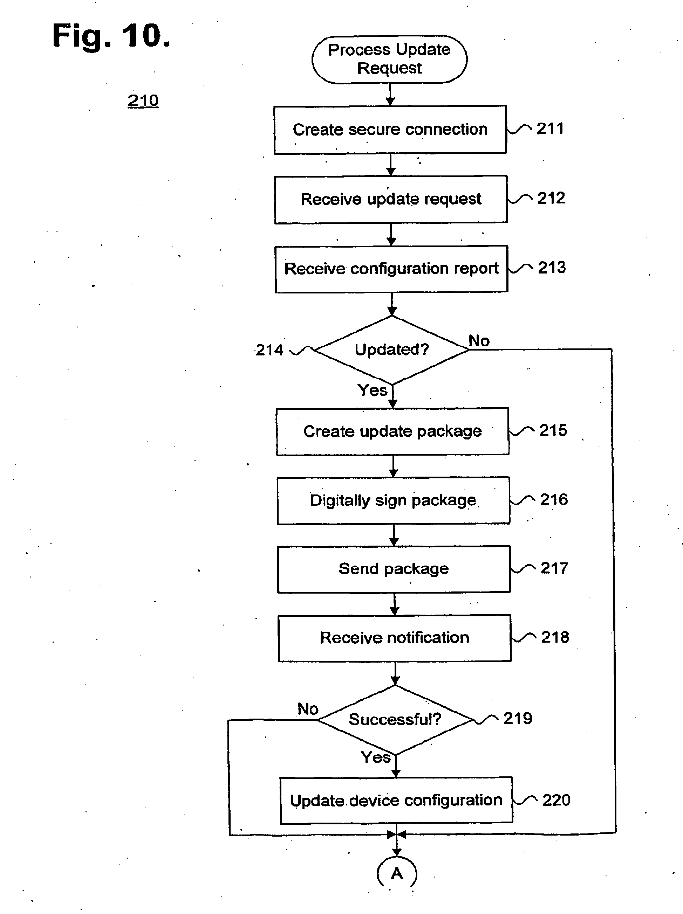

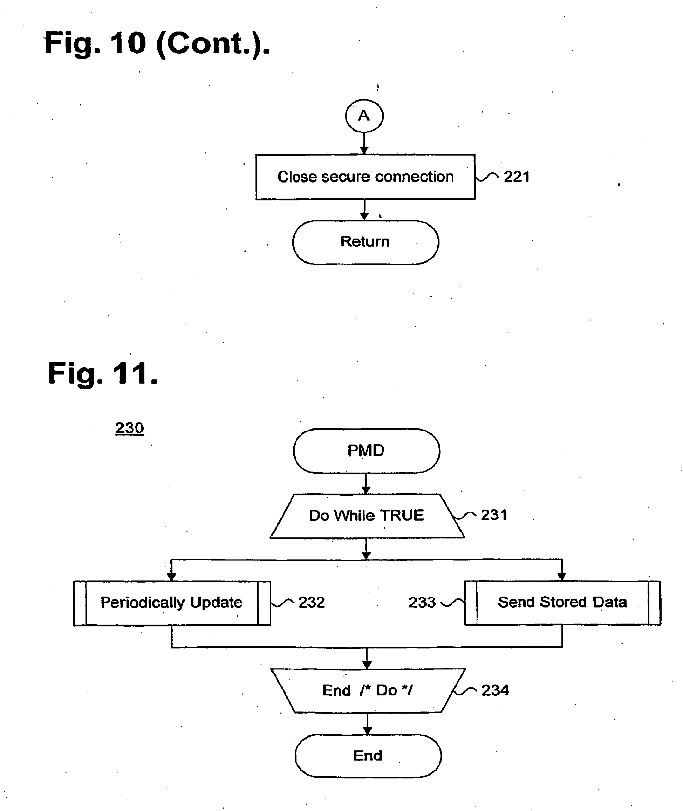

The secure distribution server 11 includes an update checker and verifier 71 that processes update requests 82 received from remotely-situated patient management devices 13. In a further embodiment, the update checker and verifier 71 processes configuration catalog updates 81 received from patient management devices and, in a further embodiment, devices, to update the configuration catalog 76 recording operational characteristics. In a still further embodiment, the update checker and verifier 71 periodically requests configuration catalog updates 81 from the patient management devices and devices. Similarly, update requests 82 can originate directly from a medical device. The update checker and verifier 71 accesses the configuration catalog 76 and identifies any feature sets 77 that are modified or new relative to each stored device configuration. The secure distribution server 11 also includes authentication 72, which packages any modified or new feature sets 77 into digitally signed packages using a stored asymmetric private key 74 unique to the secure distribution server 11. Each package is either already digitally signed by a trusted source or can be digitally signed by the secure distribution server using the asymmetric private key 74 and an asymmetric public key for that specific patient management device 13. The digitally signed feature sets are then sent to the requesting patient management device 13 or, in a further embodiment, a requesting device, as update packages 84. In a further embodiment, the digitally signed feature sets are on-demand or incrementally sent to the patient management device 13 or, in a still further embodiment, devices, as update packages 84 as modified or new feature sets 77 become available, or by unilaterally broadcasting the updated features to a certain class of devices, such as patient management devices. In addition, the update checker and verifier 71 receives notifications 80 from requesting patient management devices 13 that confirm the successful installation of feature sets 77 and updates the configuration catalog 76. The operations performed by the update checker and verifier 71 and authentication 72 are further described below with reference to FIGURE 10.

In a further embodiment, the secure distribution server 11 also includes data retrieval, analysis and storage 73. Periodically, the secure distribution server sends securely a data request 85 to one or more patient management devices 13 to request the upload of data sets 83 of stored data, which the patient management device has collected or from the one or more associated medical devices. The data sets 83 can include physiological quantitative and quality of life qualitative measures for an individual patient collected and processed in conjunction with, by way of example, an implantable medical device, such a pacemaker, ICD, or similar device; an external medical device, such as an electrocardiograph, Holter monitor or similar device; or through conventional medical testing and evaluation. As well, the data sets 83 can be analyzed against one or more medical conditions, such as described in related, commonly-owned U.S. Patent No. 6,336,903, to Bardy, issued January 8, 2002; U.S. Patent No. 6,368,284, to Bardy, issued April 9, 2002; U.S. Patent No. 6,398,728, to Bardy, issued June 2, 2002; U.S. Patent No. 6,411,840, to Bardy, issued June 25, 2002; and U.S. Patent No. 6,440,066, to Bardy, issued August 27, 2002. Finally, the data sets can be stored into a database 78 as retrieved device data 79. The database 78 need not be directly coupled to the secure distribution server 11 and can be instead remotely accessed through, for instance, a centralized database server (not shown).

In one embodiment, the secure distribution server 11 is a general-purpose server-grade computer, executing a set of software modules defined as secure distribution server software and having components conventionally found in a computer, such as, for example, a central processing unit (CPU), memory, disk storage, network interfaces, display, CD-ROM, keyboard, mouse, and various components for interconnecting these elements.

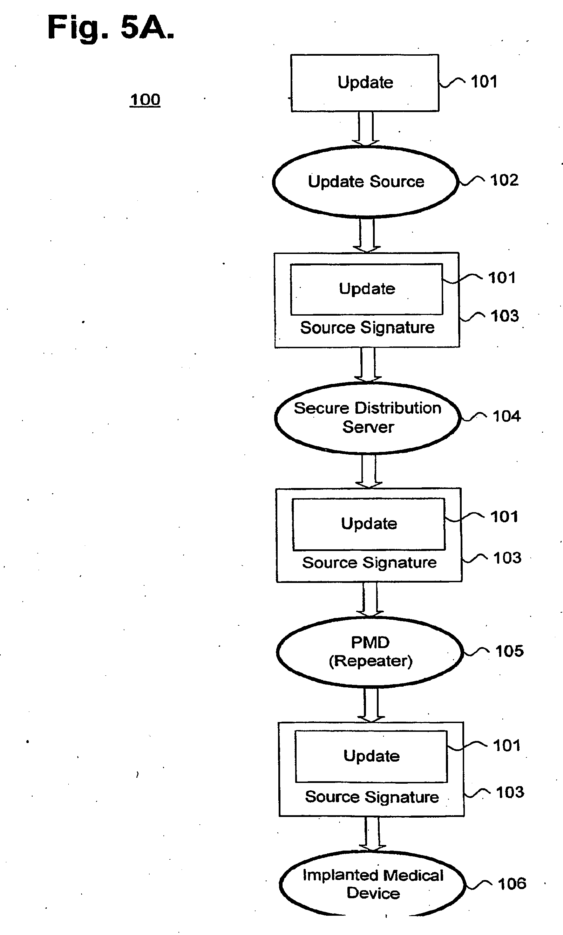

FIGURES 5A-B are routing diagrams showing end-to-end secure package processing 100, 120 by the system 10 of FIGURE 1. End-to-end processing involves a secure distribution server and requesting patient management device, which are at the end points of the network infrastructure over which update packages are securely distributed. While in transit, an update package is encapsulated in a "secure digital container" or package that was generated under the digital signature of the source of the update or a secure distribution server.

Referring first to FIGURE 5A, an update source 102 prepares and digitally signs an update package 101, which is dispatched to a secure distribution server 104 as a signed update 103. The secure distribution server source 104 authenticates and checks the integrity of the received signed update 103 before storing the signed update 103. When requested, the secure distribution server 104 dispatches the signed update 103 to a requesting patient management device 105, which also authenticates and checks the integrity of the received signed update 103 before storing or installing the update 101. In a further embodiment, the patient management device 105 dispatches the signed update 103 to an IMD 106, which similarly authenticates and checks the integrity of the received signed update 103 before installing the update 101.

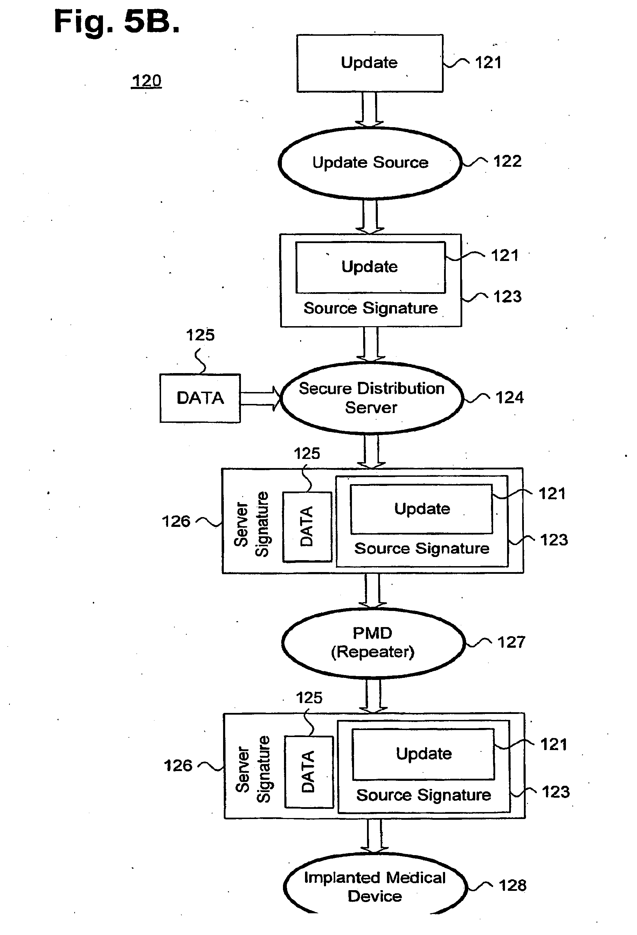

Referring next to FIGURE 5B, an update source 122 prepares and digitally signs an update package 121, which is dispatched to a secure distribution server 124 as a signed update 123. The secure distribution server source 124 authenticates and checks the integrity of the received signed update 123 before storing the signed update 123. The secure distribution server 124 also adds data 125 to the signed update 123 and digitally signs the entire combined package 126. When requested, the secure distribution server 124 dispatches the signed combined package 126 to a requesting patient management device 127, which also authenticates and checks the integrity of the received signed combined package 126 before storing or installing the update 121 and data 125. In a further embodiment, the patient management device 127 dispatches the signed combined package 126 to an IMD 128, which similarly authenticates and checks the integrity of the received combined package 126 before installing the update 121 and data 125. Other forms of end-to-end secure package processing are possible.

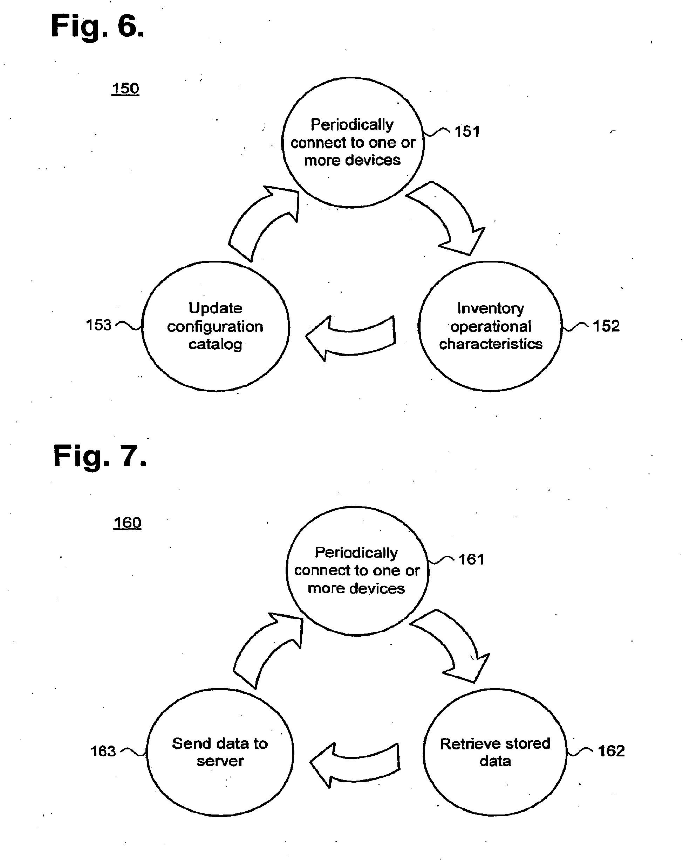

FIGURE 6 is a process flow diagram showing a configuration catalog update dialogue 150 performed by the system 10 of FIGURE 1. In one embodiment, the configuration catalog update dialogue is initiated by each patient management device 13, which periodically connects to one or more associated medical devices (operation 151) and performing an inventory of operational characteristics (block 152). The operational characteristics are then provided to the secure distribution server 11, which updates the configuration catalog 76 (block 153). The processing continues again upon the next periodic configuration catalog update (operation 151). In a further embodiment, each associated medical device periodically connects to a patient management device (operation 151) and a similar set of operations is followed to inventory operational characteristics and update the configuration catalog.

FIGURE 7 is a process flow diagram showing an upload dialogue 160 performed by the system 10 of FIGURE 1. In one embodiment, each patient management device 13 functions as a centralized hub for one or more associated medical devices by periodically connecting to one or more of the devices (operation 161) and retrieving any stored data (operation 162) collected by the medical devices. The retrieved data is then sent to the secure distribution server 11 or similar device (operation 163) for analysis and storage. The process continues upon the next periodic connection by the patient management device 13 (operation 161).

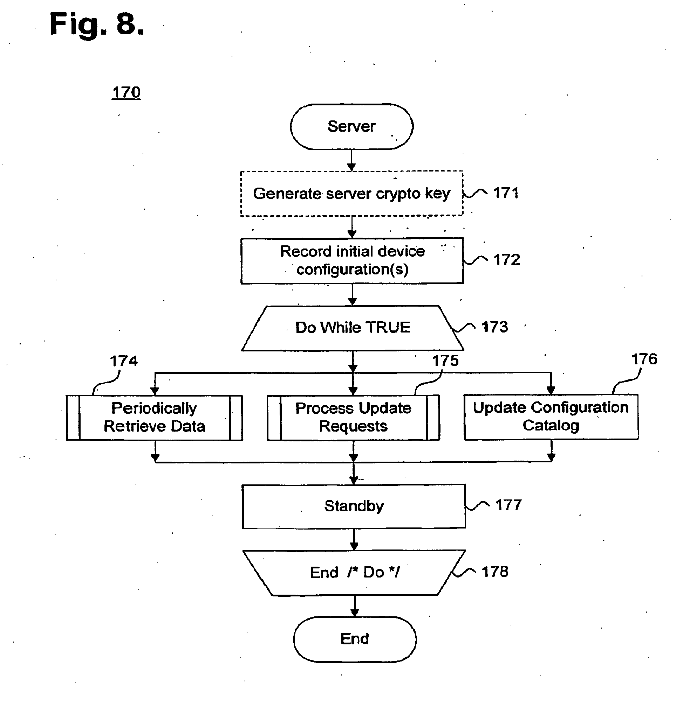

FIGURE 8 is a flow diagram showing a server method 170 for providing a secure feature set distribution infrastructure for medical device management in accordance with one embodiment. The purpose of the method is to process update requests at a secure distribution server 11 received from patient management devices 13 on a continuing basis. In a further embodiment, the method 170 also periodically retrieves data stored by the patient management devices 13.

Initially, a cryptographic key is generated (block 171). The cryptographic key is generated only once, when the server is initially configured. Depending upon the system, the cryptographic key can be generated by the secure distribution server 11 or installed as part of a manufacturing process; in either case, the cryptographic key is persistently stored by the secure distribution server 11 where the cryptographic key is subsequently used to digitally sign update packages and to establish secure connections with, for example, patient management devices. A secure connection is a communication path over which data can be exchanged without corruption, without observation of the data's content by any third party, and with assurance that the sender and receiver of the data are always known and authenticated.

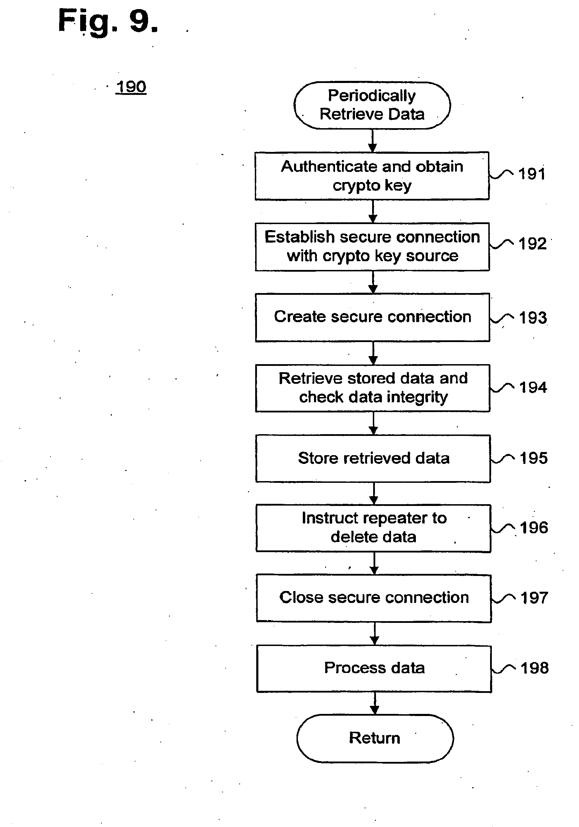

The initial device configurations of each patient management device 13 and associated medical device are recorded in the configuration catalog 76 (block 172). Update requests and, in a further embodiment, data retrievals, are processed continuously (blocks 173-178), as follows. In a further embodiment, stored data is periodically retrieved (block 174) from each patient management device 13, as further described below with reference to FIGURE 9. Similarly, update requests received from the patient management device 13 are processed (block 175), as further described below with reference to FIGURE 10. In a still further embodiment, updates of operational characteristics of each patient management device 13 and associated medical devices are recorded in the configuration catalog 76 (block 176) as provided to the secure distribution server 11, either in response to a configuration catalog update request or based on a self-generated configuration catalog update from the patient management device or devices. The secure distribution server 11 remains in a standby mode (block 177) or performs other processing when not actively retrieving data or processing update requests. Processing continues (block 178) until the secure distribution server 11 terminates operations.

FIGURE 9 is a flow diagram showing a routine 190 for periodically retrieving data for use in the method 170 of FIGURE 8. In a further embodiment, the secure distribution server. 11 or similar device periodically retrieves data collected and stored by each patient management device 13 and analyzes and stores the retrieved data into a database. This periodic data retrieval method may be initiated by either the server or the patient management device.

The server and the patient management device connect to each other over a network using a secure cryptographic method to authenticate, each to the other (block 191), to establish a shared cryptographic connection key (block 192), and to establish a cryptographically protected secure connection (block 193). The connection establishes a "session" each time a server or patient management device needs to exchange data. A single connection is established, which remains open for the duration of the session. Any data stored by the patient management device 13 is retrieved by the server and the integrity of the data is checked to ensure that no modifications occurred while the data was in transit (block 194). The data is stored into the database (block 195) and the server instructs the patient management device 13 to delete the data (block 196). The secure connection is then closed (block 197) and the retrieved data can be further processed by the secure distribution server 11 (block 198), as further described above with reference to FIGURE 8.

FIGURE 10 is a flow diagram showing a routine 210 for processing an update request for use in the method 170 (block 175) of FIGURE 8. The purpose of this routine is to process update requests received from each patient management device 13.

A secure connection with the requesting patient management device 13 is created (block 211) and an update request 82 is received (block 212). The connection establishes a "session" each time a server or patient management device needs to exchange data. A single connection is established, which remains open for the duration of the session. In a further embodiment, a non-secure connection could be used if data confidentiality were not a concern. A configuration report is received from the requesting patient management device 13 (block 213) and the configuration catalog is checked for updates (block 214). If the program code for any of the software or firmware has been updated (block 214), an update package is created (block 215) and digitally signed for the requesting patient management device 13 (block 216) using the digital signature 74 for the secure distribution server 11 (shown in FIGURE 4). In a further embodiment, the update package is already digitally signed and the secure distribution server 11 only retrieves the update package from storage 14. In a still further embodiment, the secure distribution server 11 on-demand or incrementally provides any modified or new firmware or software to the patient management device 13 as such updates become available, or by unilaterally broadcasting the updates to certain class of devices, such as patient management devices. The digitally signed package is sent to the requesting patient management device 13 or, in a further embodiment, one or more of the medical devices (block 217) and the secure distribution server 11 awaits receipt of notification of successful install (block 218). If successful (block 219), the device configuration in the configuration catalog 76 is updated (block 220). The secure connection is then closed (block 221).

In the absence of failure conditions affecting the connection between the patient management device 13 and the secure distribution server 11, the new or modified feature sets and acknowledgement notifications are communicated over a connection that is assumed to be reliable. However, error conditions, such as corrupted or lost data, can be handled by introducing error detecting and correcting functionality into the internetwork 12, either in addition to or in lieu of the error detection and correction provided by the lower layers of the network protocols implemented by the internetwork 12. For example, in one embodiment, the internetwork 12 is based on the Transmission Control Protocol/Internet Protocol (TCP/IP) network communication specification, which guarantees reliable message transport. Other network implementations are possible. For instance, the User Datagram Protocol (UDP) could be employed instead of TCP, at the cost of guaranteed data delivery, relying instead on upper protocol layers to provide the necessary error detection and correction. Similarly, other network topologies and arrangements are possible.

FIGURE 11 is a flow diagram showing a method 230 for providing a secure feature set distribution infrastructure for medical device management in accordance with one embodiment. The purpose of the patient management device method is to update the program code for the software and firmware installed on each associated medical device, as well as on the patient management device itself. In a further embodiment, each patient management device 13 collects and stores data from each of the associated medical devices for subsequent retrieval by the secure distribution server 11 or similar device.

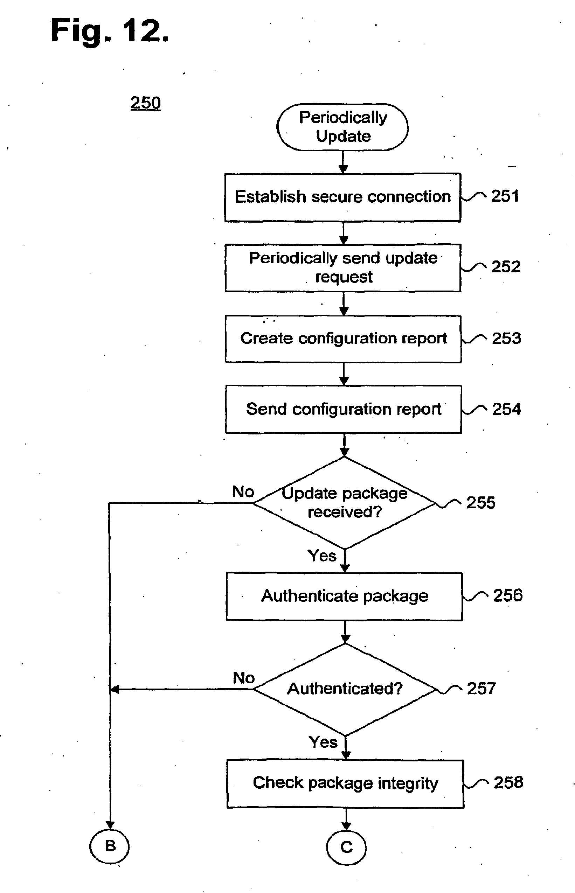

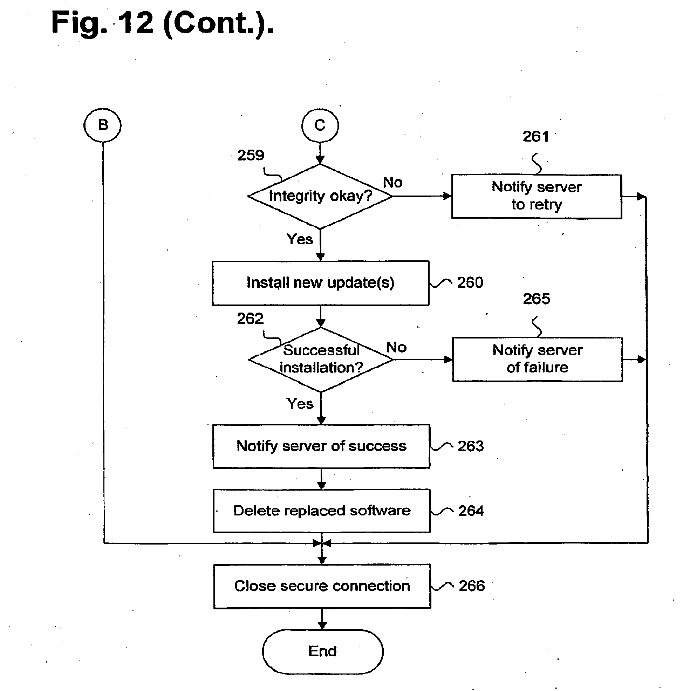

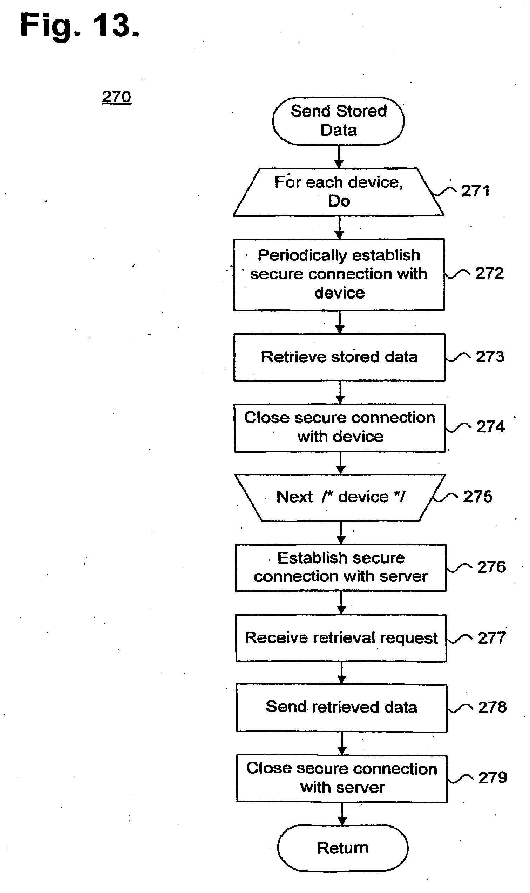

The program code for the software and firmware is periodically updated and, in a further embodiment, stored data sent, in a continuous processing loop (blocks 231-234), as follows. The program code for the firmware and software is periodically updated (block 232), as further described below with reference to FIGURE 12. In a further embodiment, data collected and stored from each associated medical device is sent to the secure distribution server 11 or similar device (block 233), as further described below with reference to FIGURE 13.

FIGURE 12 is a flow diagram showing a routine 250 for performing a periodic update for use in the method 230 of FIGURE 11. The purpose of this routine is to periodically request and install an update of the program code for the firmware and software in each associated medical device, as well as in a requesting patient management device 13 itself.

A secure connection with the secure distribution server 11 is established (block 251). The connection establishes a "session" each time a server or patient management device needs to exchange data. A single connection is established, which remains open for the duration of the session. An update request 82 is periodically sent to the secure distribution server 11 (block 252). The configuration report for each of the associated medical devices and the requesting patient management device 13 is created (block 253) and sent to the secure distribution server 11 over the secure connection (block 254). If an update package 84 is received (block 255), the package is authenticated (block 256). Otherwise, if no update package is received (block 255), the secure connection with the secure distribution server 11 is closed (block 266). If successfully authenticated (block 257), the integrity of the package is checked (block 258). Otherwise, if the authentication fails (block 257), the secure connection with the secure distribution server 11 is closed (block 266). If the integrity is sound (block 259), each update included in the package is installed (block 260). Otherwise, if the integrity is corrupt (block 259), the server is notified to retry the update request (block 261). If successful installation (block 262), the secure distribution server 11 is notified (block 263) and the replaced program code for the software or firmware is deleted (block 264). Otherwise, if installation is not successful (block 262), the server is notified of the failure (block 265). Finally, the secure connection with the secure distribution server 11 is closed (block 266). In a further embodiment, one or more of the medical devices, rather than a patient management device 13, establishes a secure connection with the secure distribution server 11 and receives, authenticates, and checks the integrity of, and installs the update packages 84. In a still further embodiment, packages 84 can be unilaterally broadcast from the secure distribution server 11 to update a certain class of devices, such as patient management devices, and each such update is installed automatically or, at the next appropriate opportunity.

In a still further embodiment, the patient management device can receive and store updates for classes of devices with which the patient management device communicates for subsequent transfer to the devices and the devices will then apply the updates.

FIGURE 13 is a flow diagram showing a routine 270 for sending stored data for use in the method 230 of FIGURE 11. In a further embodiment, the purpose of this routine is to collect and temporarily store data from each associated medical device for subsequent retrieval by the secure distribution server 11 or similar device.

Initially, each device is polled in a processing loop (blocks 271-275), as follows. A secure connection is periodically established with each medical device (block 272). Any data stored since the last secure connection is retrieved (block 273) and the secure connection is closed (block 274). Periodically, the secure distribution server 11 or similar device establishes a secure connection with the patient management device 13 (block 276). The connection establishes a "session" each time a server or patient management device needs to exchange data. A single connection is established, which remains open for the duration of the session. The patient management device 13 receives a retrieval request from the secure distribution server 11 or similar device (block 276) and the retrieved data is sent (block 278). Finally, the secure connection with the secure distribution server 11 or similar device is closed (block 279).

In a further embodiment, one or more of the devices initiates an upload of temporarily stored data to the patient management device 13, secure distribution server 11, or similar device. The device can initiate the upload according to a predefined schedule or could employ polling by the receiving system. Other forms of data upload and exchange are possible, including combinations of push, pull, and scheduled data exchange.

## Claims

1. A system (10) for providing a secure feature set (77) distribution infrastructure for medical device (18) management, comprising:
a unique association map (40) for data download between a medical device (18) and a communications device (13) transiently coupleable to the medical device (18);
a configuration catalog (76) comprising operational characteristics (41-48) of at least one of the medical device (18) and the communications device (13);
an update checker (71) to periodically check the operational characteristics (41-48) as maintained in the configuration catalog (76) against a database (75) storing downloadable sets of features (77) and to identify one or more feature sets (77) comprising changed operational characteristics (41-48) for distribution; and
a chain of trust originating with an update source (122) and terminating at the communications device (13), comprising:
an update package (123) comprising the one or more feature sets (77) digitally signed with a source signature by the update source (122) prior to storage on the database (75);
a trusted source (124) to authenticate and check the integrity of the update package (123), to digitally sign the update package upon successful authentication creating a combined package (126), and to provide the combined package to the communications device (13) over a network (12);
an authenticator (127) to authenticate and check the integrity of the combined package (126) upon receipt by the communications device (13), and
an installer to update (81) the at least one of the medical device (18) and the communications device (13) with the one or more feature sets (77) upon successful authentication of the combined package (126).

2. A system (10) according to Claim 1, wherein the trusted source (11) is a secure distribution server (11)..

3. A system (10) according to Claim 1, wherein the one or more feature sets (77) are sent to the communications device (13) in response to a download request.

4. A system (10) according to Claim 1, wherein the one or more feature sets (77) are sent on-demand or incrementally or are unilaterally broadcast from a secure distribution server (11) to the communications device (13).

5. A system (10) according to Claim 1, further comprising at least one of:
a notifier to send a notification (80) to the trusted source (11) following successful updating (81);
a collector to regularly collect physiological measures (83) from at least one of the medical device (18) into the communications device (13), and to provide the collected physiological measures (83) in response to an upload request (82) periodically received;
a further map (40) to map associations for data upload (85) between at least one further medical device (18) and the communications device (13) transiently coupleable to the at least one further medical device (18); and
a further map (40) to map unique associations for data download between a plurality of medical devices (18) and the communications device (13) transiently coupleable to each such medical device (18).

6. A system (10) according to Claim 1, wherein the one or more feature sets (77) comprises program code comprising at least one of a firmware and a software update (81).

7. A system (10) according to Claim 1, wherein the medical device (18) comprises at least one of:
an implantable medical device (18) and an external medical device (20);
a pacemaker, implantable cardioverter-defibrillator, cardiac resynchronization device, neurological implant, heart monitor, respiratory monitor, automated external defibrillator, Holter monitor, pill dispenser, weight scale, and blood pressure monitor; and
a patient communications device (13), repeater, programmer, and programmer/recorder.

8. A method (230) for providing a secure feature set (77) distribution infrastructure for medical device (18) management, comprising:
mapping (40) a unique association for data download between a medical device (18) and a communications device (13) transiently coupleable to the medical device (18);
maintaining a configuration catalog (76) comprising operational characteristics (41-48) of at least one of the medical device (18) and the communications device (13);
periodically checking the operational characteristics (41-48) as maintained in the configuration catalog (76) against a database (75) storing downloadable sets of features (77) and identifying one or more feature sets (77) comprising changed operational characteristics (41-48) for distribution;
providing a chain of trust originating with a update source (122) and terminating at the communications device (13), comprising:
generating an update package (123) comprising the one or more feature sets (77) digitally signed with a source signature by the update source (122) prior to storage on the database (75);
authenticating and checking the integrity of the update package;
digitally signing the update package with a server signature by the trusted source (11) upon successful authentication creating a combined package (126);
providing the combined package to the communications device (13) over a network (12);
authenticating and checking the integrity of the combined package (126) upon receipt by the communications device (13); and
updating (81) the at least one of the medical device (18) and the communications device (13) with the one or more feature sets (77) upon successful authentication of the combined package (126).

9. A method (230) according to Claim 8, wherein the trusted source (11) is a secure distribution server (11).

10. A method (230) according to Claim 8, further comprising:
sending the one or more feature sets (77) to the communications device (13) in response to a download request.

11. A method (230) according to Claim 8, further comprising:
on-demand or incrementally sending or unilaterally broadcasting the one or more feature sets (77) from a secure distribution server (11) to the communications device (13).

12. A method (230) according to Claim 8, further comprising at least one of:
sending a notification (80) to the trusted source (11) following successful updating (81);
regularly collecting physiological measures (83) from at least one of the medical device (18) into the communications device (13);
providing the collected physiological measures (83) in response to an upload request (82) periodically received;
mapping (40) associations for data upload (85) between at least one further medical device (18) and the communications device (13) transiently coupleable to the at least one further medical device (18); and
mapping (40) unique associations for data download between a plurality of medical devices (18) and the communications device (13) transiently coupleable to each such medical device (18).

13. A method (230) according to Claim 8, wherein the one or more feature sets (77) comprises program code comprising at least one of a firmware and a software update (81).

14. A method (230) according to Claim 8, wherein the medical device (18) comprises at least one of:
an implantable medical device (18) and an external medical device (20);
a pacemaker, implantable cardioverter-defibrillator, cardiac resynchronization device, neurological implant, heart monitor, respiratory monitor, automated external defibrillator, Holter monitor, pill dispenser, weight scale, and blood pressure monitor; and
a patient communications device (13), repeater, programmer, and programmer/recorder.

## Patentansprüche

1. System (10) zum Bereitstellen einer Verteilungsinfrastruktur für einen sicheren Merkmalsatz (77) zur Verwaltung eines medizinischen Geräts (18), wobei das System Folgendes umfasst:
eine eindeutige Zuordnungsabbildung (40) zum Datendownload zwischen einem medizinischen Gerät (18) und einem Kommunikationsgerät (13), das vorübergehend mit dem medizinischen Gerät (18) gekoppelt werden kann,
einen Konfigurationskatalog (76), der Betriebscharakteristika (41-48) wenigstens einer der Komponenten medizinisches Gerät (18) und Kommunikationsgerät (13) umfasst,
ein Aktualisierungsprüfprogramm (71), um periodisch die Betriebscharakteristika (41-48), wie sie in dem Konfigurationskatalog (76) verwaltet werden, gegenüber einer Datenbank (75) zu prüfen, die downloadbare Sätze von Merkmalen (77) speichert, und einen oder mehrere Merkmalsätze (77) zu identifizieren, die veränderte Betriebscharakteristika (41-48) zur Verteilung umfassen, und
eine Vertrauenskette, die mit einer Aktualisierungsquelle (122) beginnt und an dem Kommunikationsgerät (13) endet, wobei sie Folgendes umfasst:
ein Aktualisierungspaket (123), das den einen oder die mehreren Merkmalsätze (77) umfasst, vor dem Speichern in der Datenbank (75) durch die Aktualisierungsquelle (122) digital mit einer Quellensignatur signiert,
eine vertrauenswürdige Quelle (124) zum Authentifizieren und Prüfen der Integrität des Aktualisierungspakets (123), um das Aktualisierungspaket nach erfolgreicher Authentifizierung digital zu signieren, was ein kombiniertes Paket (126) erzeugt, und um das kombinierte Paket über ein Netz (12) dem Kommunikationsgerät (13) bereitzustellen,
einen Authentifikator (127) zum Authentifizieren und Prüfen der Integrität des kombinierten Pakets (126) nach dem Empfang durch das Kommunikationsgerät (13) und
ein Installationsprogramm zum Aktualisieren (81) der wenigstens einen der Komponenten medizinisches Gerät (18) und Kommunikationsgerät (13) mit dem einen oder den mehreren Merkmalsätzen (77) nach erfolgreicher Authentifizierung des kombinierten Pakets (126).

2. System (10) nach Anspruch 1, wobei die vertrauenswürdige Quelle (11) ein sicherer Verteilungsserver (11) ist.

3. System (10) nach Anspruch 1, wobei der eine oder die mehreren Merkmalsätze (77) als Reaktion auf eine Downloadanforderung zu dem Kommunikationsgerät (13) gesendet werden.

4. System (10) nach Anspruch 1, wobei der eine oder die mehreren Merkmalsätze (77) auf Anforderung oder inkrementell gesendet werden oder einseitig von einem sicheren Verteilungsserver (11) zu dem Kommunikationsgerät (13) übertragen werden.

5. System (10) nach Anspruch 1, das ferner wenigstens eine der folgenden Komponenten umfasst:
ein Benachrichtigungsprogramm zum Senden einer Benachrichtigung (80) an die vertrauenswürdige Quelle (11) anschließend an eine erfolgreiche Aktualisierung (81),
ein Sammelprogramm zum regelmäßigen Sammeln von physiologischen Messungen (83) aus wenigstens einem der medizinischen Geräte (18) in das Kommunikationsgerät (13) und zum Bereitstellen der gesammelten physiologischen Messungen (83) als Reaktion auf eine periodisch empfangene Uploadaufforderung (82),
eine weitere Abbildung (40) zum Abbilden von Zuordnungen für einen Datenupload (85) zwischen wenigstens einem weiteren medizinischen Gerät (18) und dem Kommunikationsgerät (13), das zeitweilig mit dem wenigstens einen weiteren medizinischen Gerät (18) gekoppelt werden kann, und
eine weitere Abbildung (40) zum Abbilden von eindeutigen Zuordnungen für einen Datendownload zwischen mehreren medizinischen Geräten (18) und dem Kommunikationsgerät (13), das zeitweilig mit jedem solchen medizinischen Gerät (18) gekoppelt werden kann.

6. System (10) nach Anspruch 1, wobei der eine oder die mehreren Merkmalsätze (77) Programmcode umfassen, der wenigstens eine der Komponenten Firmware und Softwareaktualisierung umfasst.

7. System (10) nach Anspruch 1, wobei das medizinische Gerät (18) wenigstens eine der folgenden Komponenten umfasst:
ein implantierbares medizinisches Gerät (18) und ein externes medizinisches Gerät (20),
einen Schrittmacher, einen implantierbaren Kardioverter-Defibrillator, ein Herz-Resynchronisationsgerät, ein neurologisches Implantat, einen Herzmonitor, einen Atemmonitor, einen automatisierten externen Defibrillator, einen Holter-Monitor, einen Tablettenspender, eine Waage und einen Blutdruckmonitor und
ein Patientenkommunikationsgerät (13), einen Verstärker, ein Programmiergerät und ein Programmier/Aufzeichnungsgerät.

8. Verfahren (230) zum Bereitstellen einer Verteilungsinfrastruktur für einen sicheren Merkmalsatz (77) zur Verwaltung eines medizinischen Geräts (18), wobei das Verfahren Folgendes umfasst:
Abbilden (40) einer eindeutigen Zuordnung zum Datendownload zwischen einem medizinischen Gerät (18) und einem Kommunikationsgerät (13), das vorübergehend mit dem medizinischen Gerät (18) gekoppelt werden kann,
Verwalten eines Konfigurationskatalogs (76), der Betriebscharakteristika (41-48) wenigstens einer der Komponenten medizinisches Gerät (18) und Kommunikationsgerät (13) umfasst,
periodisches Prüfen der Betriebscharakteristika (41-48), wie sie in dem Konfigurationskatalog (76) verwaltet werden, gegenüber einer Datenbank (75), die downloadbare Sätze von Merkmalen (77) speichert, und Identifizieren eines oder mehrerer Merkmalsätze (77), die veränderte Betriebscharakteristika (41-48) zur Verteilung umfassen, und
Bereitstellen einer Vertrauenskette, die mit einer Aktualisierungsquelle (122) beginnt und an dem Kommunikationsgerät (13) endet, was Folgendes umfasst:
Erzeugen eines Aktualisierungspakets (123), das den einen oder die mehreren Merkmalsätze (77) umfasst, vor dem Speichern in der Datenbank (75) durch die Aktualisierungsquelle (122) digital mit einer Quellensignatur signiert,
Authentifizieren und Prüfen der Integrität des Aktualisierungspakets,
digitales Signieren des Aktualisierungspakets mit einer Serversignatur durch die vertrauenswürdige Quelle (11) nach erfolgreicher Authentifizierung, was ein kombiniertes Paket (126) erzeugt,
Bereitstellen des kombinierten Pakets dem Kommunikationsgerät (13) über ein Netz (12),
Authentifizieren und Prüfen der Integrität des kombinierten Pakets (126) nach dem Empfang durch das Kommunikationsgerät (13) und
Aktualisieren (81) der wenigstens einen der Komponenten medizinisches Gerät (18) und Kommunikationsgerät (13) mit dem einen oder den mehreren Merkmalsätzen (77) nach erfolgreicher Authentifizierung des kombinierten Pakets (126).

9. Verfahren (230) nach Anspruch 8, wobei die vertrauenswürdige Quelle (11) ein sicherer Verteilungsserver (11) ist.

10. Verfahren (230) nach Anspruch 8, das ferner Folgendes umfasst:
Senden des einen oder der mehreren Merkmalsätze (77) zu dem Kommunikationsgerät (13) als Reaktion auf eine Downloadanforderung.

11. Verfahren (230) nach Anspruch 8, das ferner Folgendes umfasst:
Senden des einen oder der mehreren Merkmalsätze (77) auf Anforderung oder inkrementell oder einseitiges Übertragen desselben von einem sicheren Verteilungsserver (11) zu dem Kommunikationsgerät (13).

12. Verfahren (230) nach Anspruch 8, das ferner wenigstens einen der folgenden Schritte umfasst:
Senden einer Benachrichtigung (80) an die vertrauenswürdige Quelle (11) anschließend an eine erfolgreiche Aktualisierung (81),
regelmäßiges Sammeln von physiologischen Messungen (83) aus wenigstens einem der medizinischen Geräte (18) in das Kommunikationsgerät (13),
Bereitstellen der gesammelten physiologischen Messungen (83) als Reaktion auf eine periodisch empfangene Uploadaufforderung (82),
Abbilden (40) von zuordnungen für einen Datenupload (85) zwischen wenigstens einem weiteren medizinischen Gerät (18) und dem Kommunikationsgerät (13), das zeitweilig mit dem wenigstens einen weiteren medizinischen Gerät (18) gekoppelt werden kann, und
Abbilden (40) von eindeutigen Zuordnungen für einen Datendownload zwischen mehreren medizinischen Geräten (18) und dem Kommunikationsgerät (13), das zeitweilig mit jedem solchen medizinischen Gerät (18) gekoppelt werden kann.

13. Verfahren (230) nach Anspruch 8, wobei der eine oder die mehreren Merkmalsätze (77) Programmcode umfassen, der wenigstens eine der Komponenten Firmware und Softwareaktualisierung (81) umfasst.

14. Verfahren (230) nach Anspruch 8, wobei das medizinische Gerät (18) wenigstens eine der folgenden Komponenten umfasst:
ein implantierbares medizinisches Gerät (18) und ein externes medizinisches Gerät (20),
einen Schrittmacher, einen implantierbaren Kardioverter-Defibrillator, ein Herz-Resynchronisationsgerät, ein neurologisches Implantat, einen Herzmonitor, einen Atemmonitor, einen automatisierten externen Defibrillator, einen Holter-Monitor, einen Tablettenspender, eine Waage und einen Blutdruckmonitor und
ein Patientenkommunikationsgerät (13), einen Verstärker, ein Programmiergerät und ein Programmier/Aufzeichnungsgerät.

## Revendications

1. Système (10) pour fournir une infrastructure de distribution d'ensemble de caractéristiques sécurisées (77) destiné à la gestion de dispositifs médicaux (18), comprenant :
une carte d'association unique (40) pour le téléchargement vers l'aval de données entre un dispositif médical (18) et un dispositif de communication (13) pouvant être couplé de manière transitoire au dispositif médical (18) ;
un catalogue de configuration (76) comprenant des caractéristiques opérationnelles (41-48) du dispositif médical (18) et/ou du dispositif de communication (13) ;
un vérificateur de mise à jour (71) pour vérifier périodiquement les caractéristiques opérationnelles (41-48), telles que sont tenues à jour dans le catalogue de configuration (76), par rapport à une base de données (75) stockant des ensembles téléchargeables de caractéristiques (77) et pour identifier un ou plusieurs ensembles de caractéristiques (77) comprenant des caractéristiques opérationnelles modifiées (41-48) à des fins de distribution ; et
une chaîne de confiance commençant avec une source de mise à jour (122) et se terminant au niveau du dispositif de communication (13), comprenant :
une compilation de mise à jour (123) comprenant le ou les ensembles de caractéristiques (77) signés numériquement avec une signature de source par la source de mise à jour (122) avant le stockage dans la base de données (75) ;
une source de confiance (124) pour authentifier et vérifier l'intégrité de la compilation de mise à jour (123), pour signer numériquement la compilation de mise à jour lors de l'authentification réussie créant une compilation combinée (126), et pour fournir la compilation combinée au dispositif de communication (13) par le biais d'un réseau (12) ;
un authentificateur (127) pour authentifier et vérifier l'intégrité de la compilation combinée (126) lors de la réception par le dispositif de communication (13) ; et
un installateur pour mettre à jour (81) le dispositif médical (18) et/ou le dispositif de communication (13) avec le ou les ensembles de caractéristiques (77) lors de l'authentification réussie de la compilation combinée (126).

2. Système (10) selon la revendication 1, dans lequel la source de confiance (11) est un serveur de distribution sécurisé (11).

3. Système (10) selon la revendication 1, dans lequel le ou les ensembles de caractéristiques (77) sont envoyés par le dispositif de communication (13) en réponse à une demande de téléchargement.

4. Système (10) selon la revendication 1, dans lequel le ou les ensembles de caractéristiques (77) sont envoyés sur demande ou de manière incrémentielle ou sont diffusés unilatéralement au dispositif de communication (13) depuis un serveur de distribution sécurisé (11).

5. Système (10) selon la revendication 1, comprenant au moins un des éléments suivants :
un auteur de notification pour envoyer une notification (80) à la source de confiance (11) suite à une mise à jour réussie (81) ;
un collecteur pour collecter régulièrement des mesures physiologiques (83) du ou des dispositifs médicaux (18) dans le dispositif de communication (13), et pour fournir les mesures physiologiques collectées (83) en réponse à une demande de téléchargement vers l'amont (82) reçue périodiquement ;
une autre carte (40) pour faire correspondre des associations pour un téléchargement de données vers l'amont (85) entre au moins un autre dispositif médical (18) et le dispositif de communication (13) pouvant être couplé de manière transitoire à l'autre ou aux autres dispositifs médicaux (18) ; et
une autre carte (40) pour faire correspondre des associations uniques pour un téléchargement de données vers l'aval entre une pluralité de dispositifs médicaux (18) et le dispositif de communication (13) pouvant être couplé de manière transitoire à chacun de tels dispositifs médicaux (18).

6. Système (10) selon la revendication 1, dans lequel le ou les ensembles de caractéristiques (77) comprennent un code de programme comprenant un microprogramme et/ou une mise à jour logicielle (81).

7. Système (10) selon la revendication 1, dans lequel le dispositif médical (18) comprend au moins un des éléments parmi :
un dispositif médical implantable (18) et un dispositif médical externe (20) ;
un stimulateur cardiaque, un défibrillateur automatique implantable, un dispositif de resynchronisation cardiaque, un implant neurologique, un appareil de surveillance cardiaque, un appareil de surveillance respiratoire, un défibrillateur externe automatisé, un enregistreur ambulatoire de longue durée, un distributeur de pilules, une balance et un appareil de surveillance de pression artérielle, et
un dispositif de communication de patient (13), un répéteur, un programmateur et un programmateur / enregistreur.

8. Procédé (230) pour fournir une infrastructure de distribution d'ensembles de caractéristiques sécurisées (77) pour gérer des dispositifs médicaux (18), comprenant :
la mise en correspondance (40) d'une association unique pour un téléchargement de données vers l'aval entre un dispositif médical (18) et un dispositif de communication (13) pouvant être couplé de manière transitoire au dispositif médical (18) ;
le maintien à jour d'un catalogue de configuration (76) comprenant des caractéristiques opérationnelles (41-48) du dispositif médical (18) et/ou du dispositif de communication (13) ;
la vérification périodique des caractéristiques opérationnelles (41-48), telles qu'elles sont maintenues à jour dans le catalogue de configuration (76), par rapport à une base de données (75) stockant des ensembles téléchargeables de caractéristiques (77) et l'identification d'un ou de plusieurs ensembles de caractéristiques (77) comprenant des caractéristiques opérationnelles (41-48) modifiées à des fins de distribution ;
la fourniture d'une chaîne de confiance commençant avec une source de mise à jour (122) et se terminant au niveau du dispositif de communication (13), comprenant :
- la génération d'une compilation de mise à jour (123) comprenant le ou les ensembles de caractéristiques (77) signés numériquement avec une signature de source par la source de mise à jour (122) avant le stockage dans la base de données (75) ;
- l'authentification et la vérification de l'intégrité de la compilation de mise à jour ;
- la signature numérique de la compilation de mise à jour avec une signature de serveur par la source de confiance (11) lors de l'authentification réussie créant une compilation combinée (126) ;
- la fourniture de la compilation combinée au dispositif de communication (13) par le biais d'un réseau (12) ;
- l'authentification et la vérification de l'intégrité de la compilation combinée (126) à la réception par le dispositif de communication (13) ; et
- la mise à jour (81) du dispositif médical (18) et/ou du dispositif de communication (13) avec un ou plusieurs ensembles de caractéristiques (77) lors de l'authentification réussie de la compilation combinée (126).

9. Procédé (230) selon la revendication 8, dans lequel la source de confiance (11) est un serveur de distribution sécurisé (11).

10. Procédé (230) selon la revendication 8, comprenant en outre :
l'envoi de ou des ensembles de caractéristiques (77) au dispositif de communication (13) en réponse à une demande de téléchargement.

11. Procédé (230) selon la revendication 8, comprenant en outre :
l'envoi sur demande ou de manière incrémentielle ou la diffusion unilatérale au dispositif de communication (13) de ou des ensembles de caractéristiques (77) depuis un serveur de distribution sécurisé (11).

12. Procédé (230) selon la revendication 8, comprenant en outre :
l'envoi d'une notification (80) à la source de confiance (11) suite à une mise à jour réussie (81) ;
et/ou la collecte régulière de mesures physiologiques (83) dans le dispositif de communication (13) à partir du ou des dispositifs médicaux (18) ;
et/ou la fourniture des mesures physiologiques collectées (83) en réponse à une demande de téléchargement vers l'amont (82) périodiquement reçue ;
et/ou la mise en correspondance (40) des associations pour un téléchargement vers l'amont de données (85) entre au moins un autre dispositif médical (18) et le dispositif de communication (13) pouvant être couplé de manière transitoire à l'autre ou aux autres dispositifs médicaux (18) ;
et/ou la mise en correspondance (40) des associations uniques pour un téléchargement vers l'aval de données entre une pluralité de dispositifs médicaux (18) et le dispositif de communication (13) pouvant être couplé de manière transitoire à ces dispositifs médicaux (18).

13. Procédé (230) selon la revendication 8, dans lequel le ou les ensembles de caractéristiques (77) comprennent un code de programme comprenant un microprogramme et/ou une mise à jour logicielle (81).

14. Procédé (230) selon la revendication 8, dans lequel le dispositif médical (18) comprend au moins un des éléments parmi :
un dispositif médical implantable (18) et un dispositif médical externe (20) ;
un stimulateur cardiaque, un défibrillateur automatique implantable, un dispositif de resynchronisation cardiaque, un implant neurologique, un appareil de surveillance cardiaque, un appareil de surveillance respiratoire, un défibrillateur externe automatisé, un enregistreur ambulatoire de longue durée, un distributeur de pilules, une balance et un appareil de surveillance de pression artérielle, et
un dispositif de communication de patient (13), un répéteur, un programmateur et un programmateur / enregistreur.
